# EUROPEAN PATENT APPLICATION

(11) **EP 4 148 081 A1**
(43) Date of publication of application: **15.03.2023**
(21) Application number: 21799666.9
(22) Date of filing: 26.04.2021
(51) Int. Cl.: C08G 63/08, C07C 69/68, C07C 69/75, C07D 319/12

(54) **<SUP2/>? <SUB2/>?3?METHOD FOR PRODUCING LACTIDE FROM C-POLYOLS**

(30) Priority: 07.05.2020 UA 202002764 U
(71) Applicant: Limited Liability Company "Manufacturing Group "Techinservice", Kiev, 04114 (UA)
(72) Inventor: BREY, Vladimir Viktorovich, g. Kiev, 02002 (UA); SCHUTSKIY, Igor Valentinovich, g. Kiev, 01032 (UA); SHARANDA, Mikhail Evstafievich, g. Kiev, 03194 (UA); VARAVIN, Anatoliy Mihaylovich, g. Kiev, 02068 (UA); LEVITSKAYA, Svetlana Ivanovna, g. Kiev, 02167 (UA); MILIN, Artur Nikolaevich, g. Kiev, 02121 (UA); PRUDIUS, Svetlana Ivanovna, g. Kiev, 02192 (UA); ZINCHENKO, Aleksey Yurievich, Kievskaya obl., 08147 (UA)
(74) Representative: Benatov, Samuil Gabriel
(86) International application number: PCT/UA2021/000042
(87) International publication number: WO 2021/225564

(57) **Abstract**

A method for producing lactide from C₃-polyols that provides in the first step of the technological process the catalytic conversion of alcohol mixtures of C₃-polyols into methyl lactate on a solid catalyst in a flow-through reactor; and in the second step of the technological process - a reaction of methyl lactate transesterification into lactide by passing methyl lactate vapors over a heterogeneous oxide catalyst in the absence of a carrier gas and under reduced pressure. A solution of glycerin or propylene glycol in methanol is used as alcohol mixtures of C₃-polyols. In the first step of the technological process, granulated oxides from the following list are used as the catalyst: CuO/Al₂O₃, CuO-Cr₂O₃/Al₂O₃, CuO-SnO₂/Al₂O₃, CuO-ZnO/Al₂O₃, CuO-TiO₂/Al₂O₃, CeO₂/Al₂O₃, and in the second step, the catalytic condensation of methyl lactate vapors into lactide is carried out.

## Description

The invention relates to the chemical technology of organic substances, namely to producing lactide from C₃-polyols.

One of the modern problems on a global scale is the catastrophic pollution of our planet with plastic waste. Therefore, the current task of the chemical technology of organic substances is to replace traditional stable polymers with biodegradable materials. Such polymers include polylactide, which can degrade to carbon dioxide and water in a few months under natural conditions, unlike polyethylene, polypropylene or polystyrene, which do not degrade for decades. This is the reason why polylactide is widely used, first of all, as an ecological packaging material [1-3]. The growing demand to this polymer is mainly limited by the outdated technologies for producing lactic acid as a starting substance for the synthesis of monomeric lactide. Thus, the production of polylactide was expected to grow to only 1.2 million tons per year in 2020 [4], which is definitely not sufficient. Therefore, the search for new methods to produce lactide derived from other renewable raw materials, in particular, based on C₃-polyols - glycerin and propylene glycol, is of current importance.

To date, the main raw material for the synthesis of lactide is lactic acid, which is produced by fermentation of carbohydrates [5]. As a raw material, mainly corn grains are used.

Further, oligomer is produced from lactic acid in a first step at 140-200°C, followed by depolymerization thereof into a monomeric lactide under reduced pressure at temperatures above 200°C [6-7].

The main disadvantages of the above method of producing lactide from lactic acid include the multi-step technology, in different steps of which numerous byproducts are formed, in particular dimers and trimers of lactic acid, the use of a significant amount of water, which requires treatment, as well as duration and energy consumption of the process.

It should be noted that in recent years the single-step methods of lactide production via vapor-phase condensation of lactic acid or esters thereof - methyl and ethyl lactates - at 230°-300°C using solid catalysts were suggested [8-10]. These methods are based on higher thermostability of alkyl lactates as compared to lactic acid. Currently, these alkyl lactates are produced mainly through the esterification of lactic acid with methanol or ethanol. That is, lactic acid is still to be the precursor.

The patent [11] discloses a possibility of producing alkyl lactates from glycerin and alcohols using base catalysts. The reaction was carried out under static conditions, in an autoclave at 100°- 300°C under inert gas pressure of 10-200 atm for 1-20 h. Solid alkali - NaOH and KOH - were mainly used as a catalyst. Essentially, this is a reaction of glycerin dehydrogenation on solid bases with sequential formation of glyceral, pyruvic acid aldehyde and alkyl lactate.

The obvious disadvantage of the described method is the stationary conditions for producing alkyl lactates, so it is impossible to determine such practically important parameters of catalysts as their productivity and duration of stable operation time. However, this method can be used as an analogue for the first step of the claimed process. The corresponding Example 18, in which we performed dehydrogenation rather than oxidation of a mixture of glycerin and methanol into methyl lactate, is given below.

In 2016 we demonstrated the possibility of producing ethyl lactate from glycerin ethanol solution for the first time [12]. This vapor-phase process of oxidation of glycerin-ethanol mixture with air was carried out in a flow-through reactor at 230°-240° C, under pressure of 0.11 MPa using a supported cerium-oxide catalyst via the reaction

C₃H₈O₃ + C₂H₅OH + 1/2O₂ = C₅H₁₀O₃ + 2H₂O

At the same time 75% yield of ethyl lactate is achieved, which is of practical interest. Our additional studies demonstrated the possibility of producing ethyl lactate by oxidation of ethanol solution of propylene glycol. It was also shown that replacement of ethanol with methanol as a solvent of glycerin or propylene glycol allows to produce methyl lactate with 70-80% yield, which makes it of practical interest. Therefore, the possibility of replacement of lactic acid with C₃-polyols as a precursor for producing alkyl lactates was demonstrated. The next phase of our research focused on the development of a method for vapor-phase condensation of ethyl- and methyl lactates into lactide on solid acid TiO₂/SiO₂ or SnO₂/SiO₂ catalysts [13-15]. This problem was solved by original method using a reduced pressure, which provides a 1-2 seconds contact time of pure lactate vapor with catalyst at 240°-270°C. Such conditions provide catalyst productivity to lactide of 5-7 g/mol of lactide/(kg_{cat}/hour), which is of practical interest.

Two patents were chosen as the closest prior art for the proposed complex technical solution of the development of the unified technology of lactide production from C₃-polyols: patent No. UA 108920 (16) for the method of producing ethyl lactate, which provides the catalytic conversion of alcohol mixtures of C₃-polyols (glycerin ethanol solution) into ethyl lactate in a flow-through reactor on a solid catalyst (cerium dioxide), and patent No. UA 141885 (17) for the method of producing lactide from alkyl lactate into cyclic dimer of lactic acid (lactide) via transesterification reaction by passing alkyl lactate vapor in the absence of a carrier gas under reduced pressure over oxide catalyst TiO₂/SiO₂ and/or SnO₂/SiO₂.

The problem underlying the proposed technical solution is to develop a unified technological process for production of lactide from C₃-polyols - glycerin or propylene glycol (instead of traditional lactic acid) with increased options of using the components, including a vapor-phase process of catalytic oxidation of C₃-polyols into methyl lactate (as methanol is more accessible and cost-effective raw material than ethanol) and a vapor-phase condensation process of methyl lactate into lactide on solid acid catalysts.

The solution of the present problem is achieved by the fact that the method of producing lactide from C₃-polyols provides in the first step of the technological process the catalytic conversion of alcoholic mixture of C₃-polyol vapors in the air stream in a flow-through reactor on a solid catalyst into alkyl lactate, and in the second step of the technological process the reaction of transesterification of alkyl lactate into lactide is carried out by passing its vapors in the absence of a carrier gas under reduced pressure over the heterogeneous oxide catalyst TiO₂/SiO₂ or SnO₂/SiO₂, wherein in the first step the catalytic oxidation of alcohol mixture of C₃-polyol vapors into methyl lactate is performed at temperature of 200°C-230°C, a solution of glycerin or propylene glycol in methanol is used as alcohol mixtures, granulated oxides from the following list are used as the catalyst: CuO/Al₂O₃, CuO-Cr₂O₃/Al₂O₃, CuO-SnO₂/Al₂O₃, CuO-ZnO/Al₂O₃, CuO-TiO₂/Al₂O₃, CeO₂/Al₂O₃, with a total content of the supported oxides of 10-45 wt.%. In the second step of the technological process the catalytic transesterification of methyl lactate vapor into lactide is carried out at 240-270°C, preferably at 260°C, at a reduced pressure of 100-150 mbar.

Therefore, a two-step scheme for production of lactide from C₃-polyols is provided. In the first step methyl lactate is produced from an alcohol mixture of C₃-polyol vapors with glycerin or propylene glycol, and methanol. This vapor-phase process is carried out in a flow-through reactor by passing air-glycerin-methanol mixture of C₃-polyol vapors through a stationary layer, for example, cerium-oxide catalyst at 200°-230°C. In the second step, the alkyl lactate vapors is passed through a layer of, for example, TiO₂/SiO₂ catalyst under reduced pressure of 100-150 mbar and temperature of 240°-270°C. This method demonstrates the possibility of use of C₃-polyols instead of lactic acid to produce lactide via the overall reaction (e.g., for glycerin: 2C₃H₈O₃ + O₂ = C₆H₈O₄ + 4H₂O).

The following examples demonstrate the possibility of producing methyl lactate from glycerin and propylene glycol using oxide catalysts presented in the Table 1.

The following precursors were used to carry out the method.
Glycerin, C₃H₆O₃, qualification "pharm.";
propylene glycol, C₃H₈O₂, qualification "pharm.";
methanol, CH₃OH, qualification "reagent grade";
copper nitrate, Cu(NO₃)₂·3H₂O, qualification "reagent grade";
chromium nitrate, Cr(NO₃)₃·9H₂O, qualification "reagent grade";
zinc nitrate, Zn(NO₃)₂·9H₂0, qualification "reagent grade";
stannic chloride, SnCl₄·5H₂O, qualification "reagent grade";
titanium isopropoxide, C₈H₂₀O₄Ti, "Alfa Aescr";
cerium nitrate, Ce(NO₃)₃·3H₂O, qualification "chemically-pure".

Supported catalysts CuO/Al₂O₃, CuO-Cr₂O₃/Al₂O₃, CuO-SnO₂/Al₂O₃, CuO-ZnO/Al₂O₃, CuO-TiO₂/Al₂O₃ and CeO₂/Al₂O₃ were synthesized by impregnating the granular γ-Al₂O₃ with a solution of appropriate nitrates, followed by a heat treatment at 400°C, 2 h.

The textural parameters of the catalysts were obtained by means of lowtemperature nitrogen adsorption-desorption method using the Quantachrome Nova 2200e Surface Area and Pore Size Analyser (Table 1).

**Table 1.**

| Structural characteristics of the catalysts used | | | | | |
|---|---|---|---|---|---|
| **Catalyst** | **Content, wt.%** | | **S_{spec.}, m²/g** | **Vₚₒᵣₑ, cm³/g** | **Dₚₒᵣₑ, nm** |
| | **CuO** | **MeOₓ** | | | |
| CuO/Al₂O₃ | 45 | - | 160 | 0.3 | 7.5 |
| CuO-Cr₂O₃/Al₂O₃ | 45 | 8 (Cr₂O₃) | 150 | 0.3 | 7.2 |
| CuO-SnO₂/Al₂O₃ | 20 | 20 (SnO₂) | 200 | 0.3 | 7.7 |
| CuO-TiO₂/Al₂O₃ | 20 | 12 (TiO₂) | 200 | 0.3 | 7.2 |
| CuO-ZnO/Al₂O₃ | 14 | 4 (ZnO) | 160 | 0.4 | 10.6 |
| CeO₂/Al₂O₃ | - | 10 (CeO₂) | 220 | 0.65 | 4.8 |

The catalytic experiments were carried out as follows. 10 cm³ of one of the catalysts presented in the Table 1 was placed in a stainless-steel flow-through reactor with a diameter of 12 mm and a length of 120 mm.

The catalyst was placed in the middle part of the reactor. The free space above and below the catalyst was filled with an inert filler (quartz). A 20 wt.% solution of glycerin or propylene glycol in methanol was fed into the reactor using the Waters Model 950 pump with a space velocity (LHSV) of 0.5-1 h⁻¹ at a temperature of 180°C to 230°C and a pressure of 0.15 MPa. The catalyst load (L) was 1.5-3.0 mmol of glycerin or propylene glycol per 1 g of catalyst per hour. Air was directly fed into the reactor in such an amount that the molar ratio "oxygen/glycerin" was 0.5 to 1.0, and the molar ratio "oxygen/propylene glycol" was 1.0 to 2.0. The space velocity of air feed, depending on the load on the catalyst to polyols, was 80 - 170 h⁻¹.

The analysis of reaction products after 3 hours of catalyst operation was performed using gas chromatography ("Agilent 7820A") and ¹³C NMR spectroscopy ("Bruker Avance-400") methods. The identification of the reaction products was performed according to ¹³C NMR spectra using the database of organic compounds (SDBS, National Institute of Advanced Industrial Science and Technology, Japan, www.aist.gojp). The conversion of glycerin or propylene glycol and the selectivity of the reaction products were calculated from the data of the chromatographic analysis (wt.%).

The examples of conversion of glycerin and propylene glycol on oxide catalysts, supporting the possibility of implementation of the claimed method, are given below.

### Example 1.

The reaction was carried out at 210°C on a CuO-Cr₂O₃/Al₂O₃ catalyst with the feeding of a 20% glycerin solution in methanol, at a catalyst load of 1.8 mmol glycerin/(g_{cat}·h). The conversion of glycerin was 100%, the selectivity to methyl lactate was 56%, and the catalyst productivity for methyl lactate was 1.0 mmol ML/(g_{cat}·h).

### Examples 2-7.

The following examples are given for 20% glycerin-methanol solutions. The experiments corresponding to the Examples 2-7 were performed similarly to the Example 1 using CuO-Cr₂O₃/Al₂O₃ catalyst but under other conditions. The results are shown in the Table 2.

**Table 2.**

| Synthesis of methyl lactate on CuO-Cr₂O₃/Al₂O₃ catalyst under various conditions: | | | | | | | |
|---|---|---|---|---|---|---|---|
| **Example No.** | **T, °C** | **LHSV, h1** | **L, mmol Gl/(g·h)** | **O₂/Gl, mol** | **X, %** | **S, mol%** | **Y, mmol ML/ (g_{cat}·h)** |
| 3 | 220 | 0.5 | 1.8 | 0.7 | 100 | 51 | 0.9 |
| 4 | 180 | 0.5 | 1.8 | 0.7 | 92 | 47 | 0.8 |
| 5 | 200 | 1.0 | 3.6 | 0.7 | 95 | 53 | 1.9 |
| 6 | 210 | 0.8 | 3.0 | 0.7 | 99 | 54 | 1.6 |
| 7 | 200 | 0.6 | 2.2 | 0.5 | 100 | 50 | 1.1 |
| 8 | 210 | 0.6 | 2.2 | 0.9 | 100 | 48 | 1.0 |

| | | | | | | | |
|---|---|---|---|---|---|---|---|
| LHSV - space velocity of 20% glycerin solution in methanol, h⁻¹; L - catalyst load; O₂/Gl - molar ratio "oxygen/glycerin"; X - glycerin conversion, S - selectivity to methyl lactate; Y - catalyst productivity to methyl lactate. | | | | | | | |

The above examples demonstrate that the process is optimally implemented at temperatures of 200°-210° C, at a catalyst load of 1.8-2.2 mmol/g_{cat}·h, at a molar ratio of "O₂/Glycerin" within the range of 0.5-0.7. The reduction of temperature below 200°C, as well as a load increase of more than 3 mmol/(g_{cat}·h) does not provide 100% conversion of glycerin. An increase in the O₂/glycerin ratio of more than 0.7 results in a decrease in selectivity to methyl lactate.

### Examples 8-16.

The experiments corresponding to Examples 8-16 were performed similarly to the Example 1, using 20% solutions of glycerin and propylene glycol in methanol on other catalysts.

The results are summarized in the table 3.

**Table 3**

| Synthesis of methyl lactate from glycerin and propylene glycol on various catalysts | | | | | | | | |
|---|---|---|---|---|---|---|---|---|
| **Example No.** | **Catalyst** | **Polyol** | **T, °C** | **LHSV h1** | **L, mmol / (g·h)** | **X, %** | **S, %** | **Y, mmol ML /(g_{cat}·h)** |
| 8 | CuO-Cr₂O₃/Al₂O₃ | G1 | 210 | 0.6 | 2.2 | 100 | 56 | 1.2 |
| 9 | CuO-Cr₂O₃/Al₂O₃ | PG | 210 | 0.5 | 2.2 | 100 | 55 | 1.2 |
| 10 | CuO/Al₂O₃ | G1 | 210 | 0.6 | 2.2 | 100 | 55 | 1.2 |
| 11 | CuO/Al₂O₃ | PG | 200 | 0.5 | 2.2 | 100 | 54 | 1.2 |
| 12 | CuO-SnO₂/Al₂O₃ | G1 | 200 | 0.6 | 2.2 | 100 | 52 | 1.1 |
| 13 | CuO-TiO₂/Al₂O₃ | G1 | 210 | 0.6 | 2.2 | 100 | 53 | 1.1 |
| 14 | CuO-ZnO/Al₂O₃ | G1 | 210 | 0.6 | 2.2 | 100 | 51 | 1.1 |
| 15 | CeO₂/Al₂O₃ | G1 | 230 | 0.6 | 2.2 | 100 | 89 | 1.9 |
| 16 | CeO₂/Al₂O₃ | PG | 230 | 0.5 | 2.2 | 100 | 76 | 1.2 |

The above examples demonstrate that the process can be implemented on copper-containing catalysts with a composition of CuO/Al₂O₃ and CuO-MeₓO_{y}/Al₂O₃, where Me is Zn, Ti, Cr, Sn, x = 1 - 2, y = 1 - 3, as well as on the supported cerium-containing CeO₂/Al₂O catalysts.

### Example 17.

The reaction was performed at 210°C on a Cu45%-Cr8%/Al(P-192) catalyst in an inert atmosphere in the glycerin dehydrogenation mode as proposed in [11]. The catalyst was pre-activated by passing 50 ml/min of a mixture of 10% H₂ + 90% N₂, at 180 °C to 210 °C for 4 h. The catalyst load was 1.8 mmol of glycerin/(g_{cat·}h).

Nitrogen in an amount of GHSV = 150 h⁻¹ was fed into the reactor instead of air. The conversion of glycerin during the first 8 hours was 100%. After 8 h of operation, 48 g of a product having the following composition (component % wt.) was yielded:
Methanol - 66.5
Methyl lactate - 12.2
Methyl acetate - 0.5
Dimethoxymethane - 1.1
Hydroxyacetone - 3.6
Methyl formate - 0.8
Others - 8.2
Water - 7.1

The glycerin conversion was 100%, selectivity to methyl lactate was 52%, yield of methyl lactate was 52%, catalyst productivity to methyl lactate was 0.9 mmol ML/(g_{cat}·h). After 8 hours of operation the glycerin conversion started to decrease gradually and after 10 hours of operation it was 92%. That is, in the non-oxidizing environment there was rather rapid deactivation of the catalyst, and its productivity to methyl lactate was less than in the oxidation mode of glycerin-methanol mixture.

### Example 18.

The methyl lactate was produced started from a 20% methanol solution of glycerin on CuO-Cr₂O₃/Al₂O₃ catalyst under the conditions of the Example 9. After 34 hours of operation, 200 g of the product of the following composition (component wt.%) was produced:
Methanol - 68.2
Methyl lactate - 13.1
Methyl acetate - 3.3
Dimethoxymethane - 3.9
Acetoin - 0.5
Methyl formate - 3.8
Others - 0.2
Water - 7.0.

200 g of this product was distilled in a distillation apparatus with a thermostatic nozzle. The first fraction of 71 g was distilled at a condensation temperature of 52°-64°C and contained 56.3% of methanol, 10.3% of methyl acetate, 12.2% of dimethoxymethane, 11.9% of methyl formate, 1.6% of acetoin and 7.8% of water. The second fraction of 102 g at a condensation temperature of 64.4°C contained 102 g of methanol. Further, at a pressure of 25 mbar (2.5 kPa) and a condensation temperature of 88°C, 26.1 g of methyl lactate was produced. The distillation residue of 0.7 g contained tar-like substances.

The resulting methyl lactate was directed to vapor phase condensation into lactide on a supported TiO₂/SiO₂ catalyst under reduced pressure of 100 mbar at 260°C, as described in our patent [17]. In particular, the condensation reaction of methyl lactate vapor was carried out at 260°C on a TiO₂/SiO₂ catalyst containing 5% TiO₂, a catalyst load of 35 mmol ML/(g_{cat}·h) with a pressure of 100 mbar, which was generated using a vacuum pump. The contact time was 1 s. The conversion of methyl lactate was 45%, selectivity to lactide was 65%, lactide yield was 29%, catalyst productivity to lactide was 7.5 mmol l/(g_{cat}·h).

Therefore, the provided method of producing lactide, which uses instead of lactic acid a step of producing methyl and ethyl lactates from solutions of glycerin and propylene glycol (as precursors) in methanol or ethanol, using copper or cerium-derived catalysts, allows to avoid the environmentally unfavorable enzymatic process of producing lactic acid as well as the energy consuming step of depolymerization of lactic acid oligomers into lactide, which significantly reduces energy consumption when producing the desired product.

### List of references:

1. Gupta A.P., Kumar V. New emerging trends in synthetic biodegradable polymers - polylactide: a critique // Eur. Polymer J., 2007, V. 43, s. 10, P. 4053- 4074.
2. R. Auras, L.-T. Lim, S.E.M. Selke, H. Tsuji (eds). Hoboken Poly(lactic acid): synthesis, structures, properties, processing and applications., New Jersey: John Wiley & Sons, Inc., 2010. 528 p.
3. S. Farah, D. G. Anderson, R. Langer Physical and mechanical properties of PLA, and their functions in widespread applications - A comprehensive review, Adv. Drug Deliv. Rev. (2016), http://dx.doi.org/10.1 016/j.addr.2016.06.012.
4. Lactic acid and poly lactic acid (PLA) market analysis by application (packaging, agriculture, transport, electronics, textiles) and segment forecasts to 2020. Grand view research, 2014.
5. W. GROOT, JAN VAN KRIEKEN, O. SLIEKERSL, SICCO DE VOS Production and purification of lactic acid and lactide p.2-17 //Part 1 in Poly(lactic acid): Synthesis, Structures, Properties, Processing, and Applications, edited by R. Auras, L.-T. Lim, S. E. M. Selke, and H. Tsuji// 2010 John Wiley & Sons, Inc.
6. Patent US5247058 Gruber P.R., Hall E.S., Kolstad J.J., Iwen M.L., Benson R.D., Borchardt R.L. Continuous process for manufacture of lactide polymers with controlled optical purity // Publ. Date: 21.09.1993.
7. Patent US6326458, B1, Gruber P.R, Hall E.S., Kolstad J.J., Iwen M.L., Benson R.D., Borchardt R.L. Continuous process for manufacture of lactide and lactide polymers // Publ. Date: 04.12.2001.
8. Patent US5332839, Benecke H.P., Markle R.A., Sinclair R.G., Catalytic production of lactide directly from lactic acid // Publ. Date: 26.07.1994.
9. Patent US 9539561, B2, Hwang D.W., Chang J.-S., Upare P.P., Lee U-H., Hwang Y.-K., Method for producing lactide directly from lactic acid and a catalyst used therein // Publ. Date: 10.01.2017.
10. Patent EP No. 2264020, A1, Chang J.-S., Hwang Y.-K., Lee J.-H., Lee J.-M., Jung M.-H., Catalyst for direct conversion of esters of lactic acid to lactide and the method for producing lactide the same // Publ. Date: 22.12.2010.
11. Application US 2013/007954 7 (A1) Method for preparing alkyl lactate and method for preparing lactamide using the same, cl. C07C 67/40, C07C 231/02, publ. 28.03.2013.
12. A.M. Mylin, V.V. Brey Selective conversion of ethanol solution of glycerin into methyl lactate on CeO2/Al2O3 catalyst // Ukr.khim. J. 2016 v.82, No. 2, p. 79-83.
13. Varvarin A.M., Levytska S.I., Brey V.V. Vapor-phase condensation of methyl lactate into lactide on a SnO2/SiO2 catalyst. // Reports of the National Academy of Sciences of Ukraine, 2018. (1). 73-79.
14. Varvarin A.M., Levytska S.I., Brey V.V. Condensation of methyl lactate into lactide on a SnO2/SiO2 catalyst // Kataliz i nephtechimija. - 2018. No. 27. - P. 19-24.
15. Varvarin A.M., Levytska S.I., Hlushchuk Ya.R., Brey V.V. Vapor-phase condensation of lactide from ethyl lactate on a TiO2/SiO2-catalyst.// Ukr. Khim. Journal. - 2019, 85. No. 7.- P. 31-37 doi: 10.3360910041-6045.85.7.2019.31-37.
16. Patent No. UA 108920, cl. C07C 51/00, publ. 10.08.2016 "Method for producing ethyl lactate from ethanol solution of glycerin on catalyst containing cerium dioxide".
17. Patent No. UA 141885, cl. C07C69/75, publ. 27.04.2020 "Method of producing lactide from alkyl lactates".

## Claims

1. A method for producing lactide from C₃-polyols that provides in the first step of the technological process the catalytic oxidation of alcohol mixtures of C₃-polyol vapors into alkyl lactate on a solid catalyst in the air stream in a flow-through reactor, and in the second step of the technological process - a reaction of alkyl lactate transesterification into lactide by passing alkyl lactate vapors over a heterogeneous oxide catalyst TiO₂/SiO₂ or SnO₂/SiO₂ in the absence of a carrier gas and under reduced pressure, **characterized in that** in the first step the catalytic oxidation of alcohol mixtures of C₃-polyols vapors into methyl lactate is carried out at temperature of 200°C-230°C, wherein a solution of glycerin or propylene glycol in methanol is used as alcohol mixtures, and granulated oxides from the following list are used as the catalyst: CuO/Al₂O₃, CuO-Cr₂O₃/Al₂O₃, CuO-SnO₂/Al₂O₃, CuO-ZnO/Al₂O₃, CuO-TiO₂/Al₂O_{3;} CeO₂/Al₂O₃ with a total content of the supported oxides of 10-45 wt.%.

2. A method for producing lactide from C₃-polyols according to claim 1, **characterized in that** in the second step of the technological process the catalytic transesterification of methyl lactate vapors into lactide is carried out at 240-270°C, preferably at 260°C, under reduced pressure of 100-150 mbar.
